# FASCICULE DE BREVET EUROPEEN

(11) **EP 0 605 680 B1**
(45) Date de publication et mention de la délivrance du brevet: **04.09.2002**
(21) Numéro de dépôt: 93913195.9
(22) Date de dépôt: 25.06.1993
(51) Int. Cl.: C12N 15/86, C07K 14/06

(54) **MUTANTS DU VIRUS DE LA RHINOTRACHEITE INFECTIEUSE BOVINE ET VACCINS**
MUTANTEN DES INFEKTIÖSEN RINDER RHINOTRACHEITIS-VIRUS UND IMPFSTOFFE
INFECTIOUS BOVINE RHINOTRACHEITIS VIRUS MUTANTS AND VACCINES

(30) Priorité: 26.06.1992 FR 9207930
(43) Date de publication de la demande: 13.07.1994
(73) Titulaire: MERIAL, 69002 Lyon (FR)
(72) Inventeur: LEUNG-TACK, Patricia, F-69000 Villeurbanne (FR); LEGASTELOIS, Isabelle, Christine, Marie-Andrée, F-69440 Mornant (FR); AUDONNET, Jean-Christophe, Francis, F-69006 Lyon (FR); RIVIERE, Michel, Emile, Albert, F-69130 Ecully (FR)
(74) Mandataire: Bernasconi, Jean
(86) Numéro de dépôt international: FR9300642
(87) Numéro de publication internationale: WO94000586

(56) Documents cités:
- EP-A- 0 256 677
- EP-A- 0 316 658
- EP-A- 0 326 127
- EP-A- 0 431 668
- WO-A-87/02058
- WO-A-87/04463
- JOURNAL OF GENERAL VIROLOGY, vol. 71, 1990, pages 2969-2978, Reading, Berks, GB, J.-C, AUDONNET et al.: "Equine herpesvirus type 1 unique short fragment encodes glycoproteins with homology to herpes simplex virus type 1 gD, gI and gE", le document en entier (cité dans la demande)

## Description

### DOMAINE DE L'INVENTION

La présente invention a pour objet un procédé de production des protéines gE et 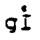 du virus BHV-1.

La présente invention décrit des segments du génome de l'herpèsviru de la rhinotrachéite infectieuse bovine (IBR), contenus dans sa région unique courte, et codant notamment pour la glycoprotéine 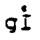 . La présente invention décrit aussi des segments d'ADN contenant le gène codant pour ces glycoprotéines antigéniques et contenant des séquences promotrices potentielles jusqu'à 400 nucléotides en 5' de chaque gène, segments qui sont utiles comme source possible de promoteurs BHV-1, pour l'expression des gènes pour produire cette glycoprotéine et d'autres, telles que gE qui peuvent à leur tour être utilisées pour induire des anticorps qui les reconnaissent.

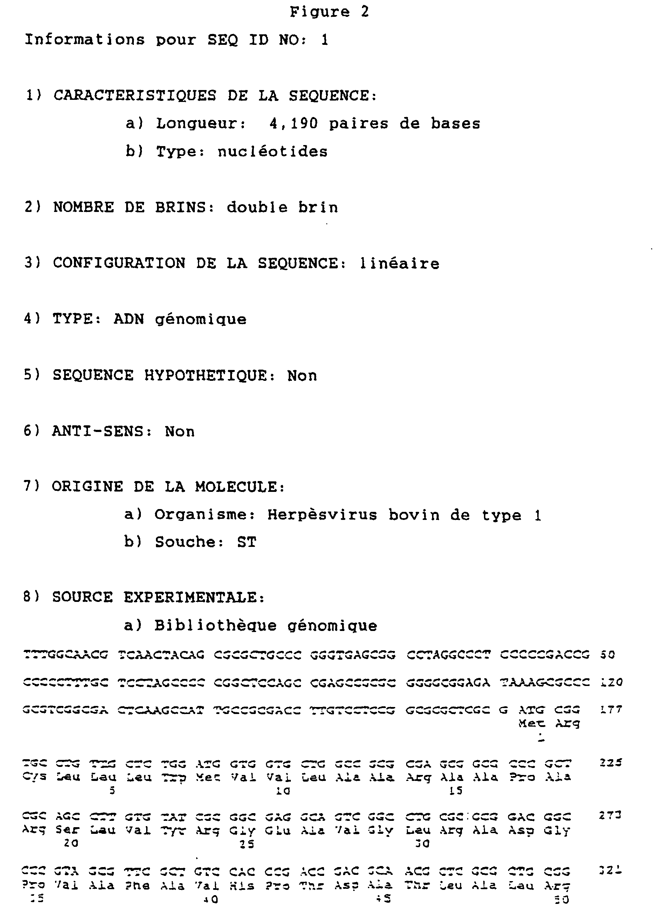

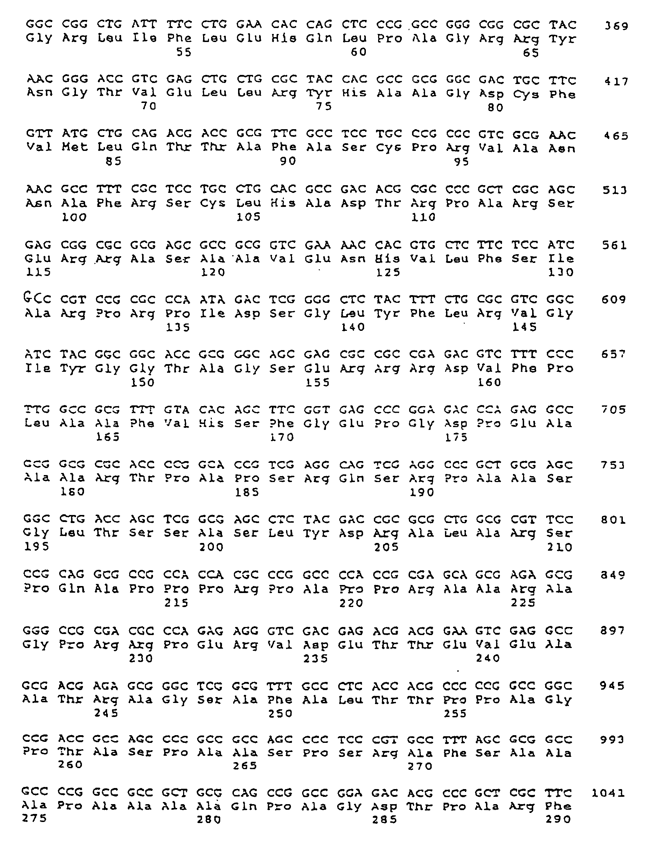

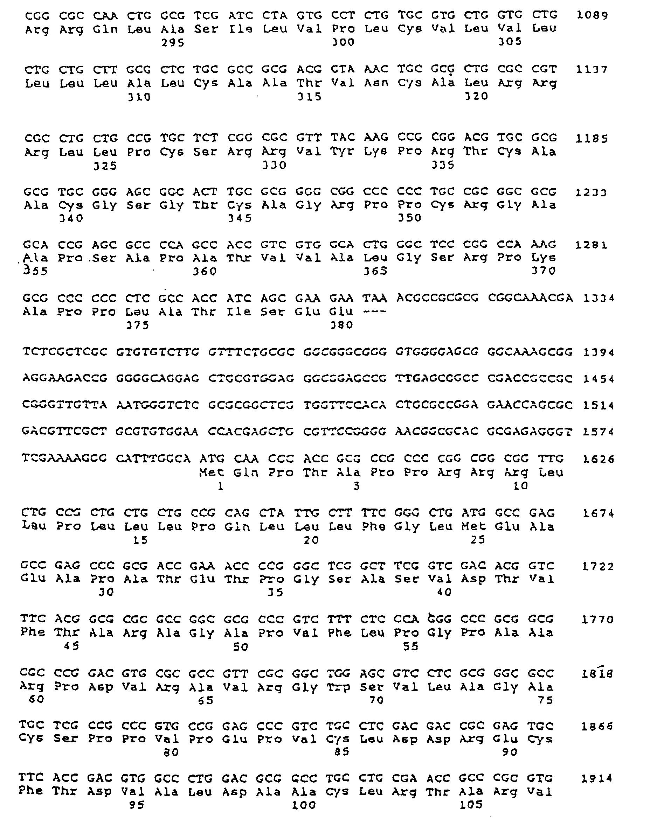

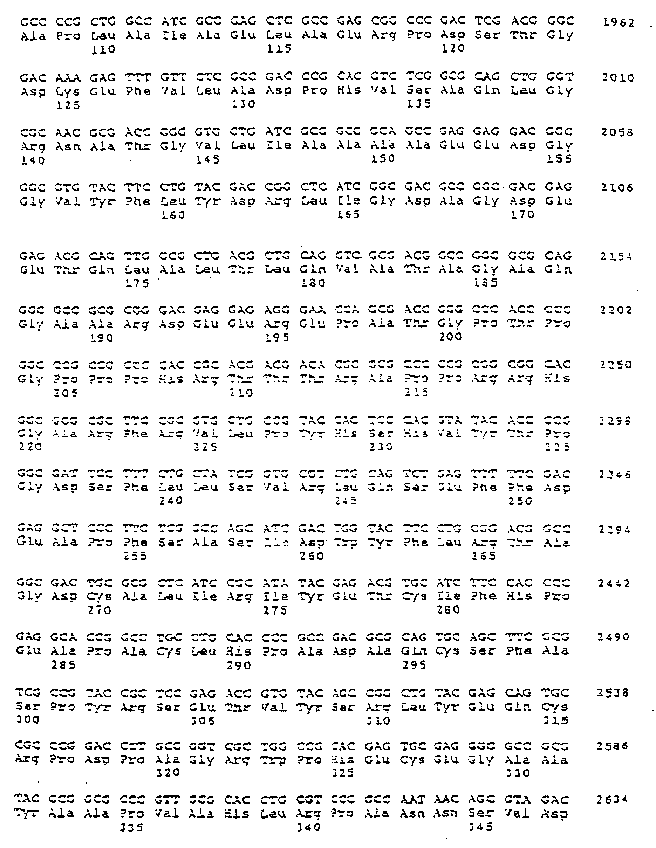

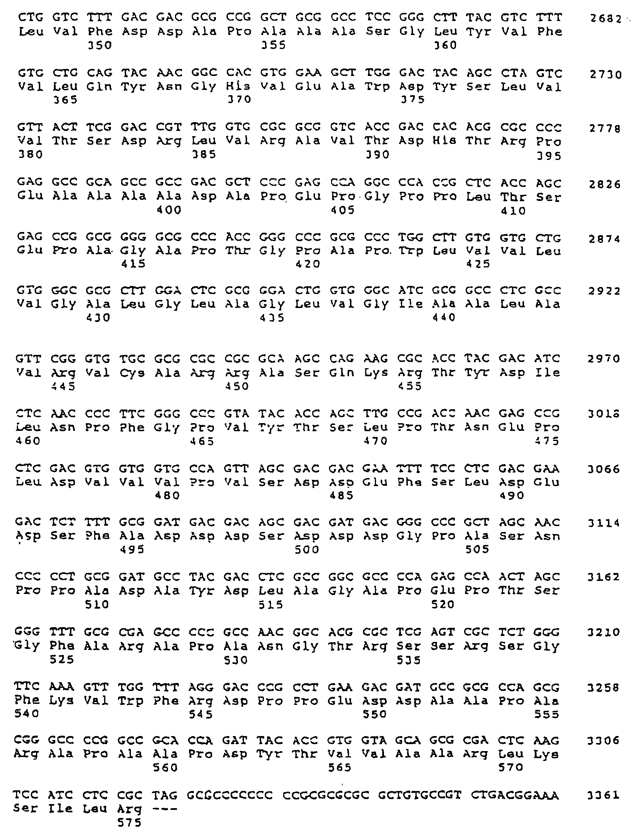

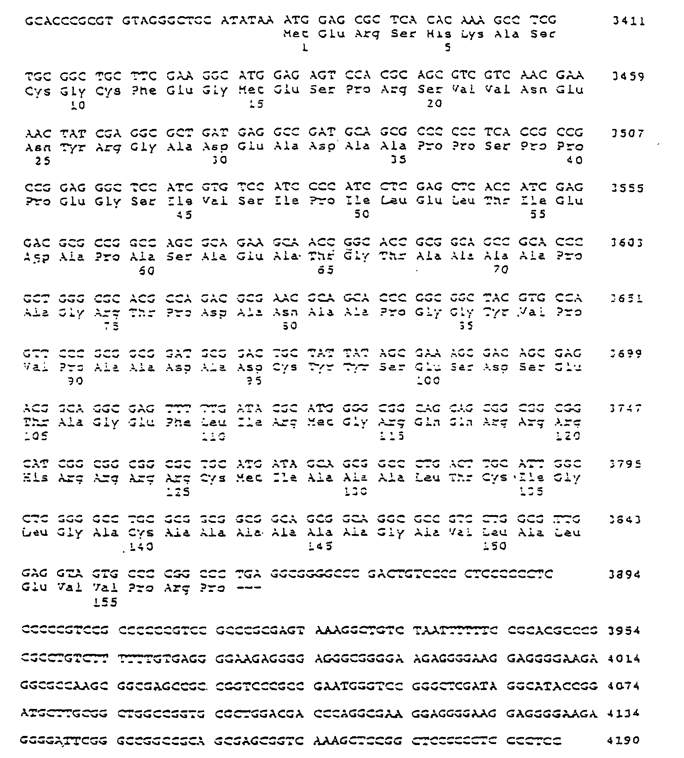

La séquence d'ADN délimitée par les nucléotides 172 et 1 311 de la séquence présentée sur la figure 2 correspond au gène codant pour 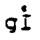 .

La séquence d'ADN délimitée par les nucléotides 1 594 et 3 318 de la séquence présentée sur la figure 2 correspond au gène codant pour gE.

La présente invention a pour objet l'expression du gène BHV-1 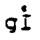 dans un système d'expression baculovirus afin d'utiliser les produits d'expression obtenus comme antigènes. La présente invention est également relative à la préparation de réactifs sérologiques à partir des antigènes BHV-1 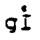 seuls ou avec les antigènes BHV-I gE.

Ces antigènes BHV-1 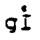 et évenuellement BHV-1 gE peuvent être utilisés pour détecter la présence d'anticorps spécifiques dans le sérum des bovins infectés par BHV-1.

La présente invention décrit une séquence d'ADN de 4 190 pb contenue dans la réqion Us du génome de BHV-1 et codant pour les qènes homologues de HSV-1 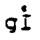 , HSV-1 gE et HSV-1 US9.

### DONNEES BIBLIOGRAPHIQUES

La rhinotrachéite infectieuse bovine (IBR) est une maladie infectieuse aiguë et contagieuse des bovins, souvent aggravée par des complications bactériennes (Pastoret P.P., Ann. Med. Vet., 122, 371-391, 1978). Cette maladie se présente, soit sous une forme respiratoire (IBR proprement dite), qui est prédominante et qui affecte principalement les jeunes bovins, soit sous une forme génitale, la vulvovaginite pustuleuse infectieuse (IPV) qui a été historiquement importante mais qui est actuellement plus rare (Yates W.P., Can. J. Comp. Med., 46, 225-263, 1982). L'IBR est l'une des causes principales des affections respiratoires des bovins et entraîne des pertes économiques considérables tant dans l'industrie laitière que dans la production de viande. Devant l'importance économique de cette maladie, des barrières sanitaires visant à interdire l'importation d'animaux infectés ont été mises en place par un certain nombre de pays.

Cette maladie est de nature virale et son agent étiologique a été identifié comme étant l'herpèsvirus bovin de type 1 (BHV-1) (Madin S.H. et al., Science, 124, 721-722, 1956). Ce virus est classé dans la famille des Herpesviridae et appartient plus précisément à la sous-famille des Alphaherpesviridae dont le prototype est le virus herpès simplex humain de type 1 (HSV-1) (Armstrong J.A. et al., Virology, 14, 276-285, 1961 ; Roizman B. et al., Arch. Virol., 123, 425-449, 1992). Comme la plupart des herpèsvirus, le virus BHV-1 peut être présent à l'état latent chez son hôte et être réactivé dans certaines circonstances (stress, transport...). Le contrôle de cette maladie repose à la fois sur une prophylaxie sanitaire et sur une prophylaxie médicale.

Le diagnostic clinique est rendu difficile dans les formes graves surinfectées, qui montrent alors des similitudes avec d'autres infections respiratoires bovines, et n'est pas toujours facile à mettre en oeuvre. La preuve formelle d'une maladie IBR ne peut être apportée qu'avec des tests de laboratoire (Gilbert Y. et al., Rev. Med. Vet., 3, 383-389, 1976 ; Fedida M. et Dannacher G., Bull. Lab. Vet., 5, 35-46, 1982). L'isolement du virus en culture de cellules à partir d'écouvillonnages réalisés sur les animaux suspects reste le meilleur moyen de diagnostic de la maladie. Mais cette technique est lente et surtout lourde à mettre en oeuvre. Aussi lui préfère-t-on désormais des techniques plus simples et plus rapides basées sur la détection des antigènes BHV-1 ou, de plus en plus, sur la mise en évidence d'anticorps spécifiques dans le sérum des animaux suspects. De nombreux tests utilisant la technique ELISA sont actuellement sur le marché (Cheng-Feng Z. et Forbes S.D., New Zealand, Vet. J., 36, 204-205, 1988). La séroneutralisation demeure toutefois la technique officielle lors des transactions commerciales européennes ou internationales.

Le contrôle de l'IBR repose principalement. sur la vaccination (Straub O.C et Mawhinney I.C., Vet. Rec., 122, 407-411, 1988).

Trois types de vaccins ont été utilisés pour protéger les bovins contre l'IBR (Kit M. et al., European Patent Application n° 0316 658 A1, 1988).
- Les vaccins à base de virus atténués obtenus par un grand nombre de passages successifs des virus pathogènes sur des cellules d'origine bovine, par adaptation de ces mêmes virus sur des cellules d'origine autre que bovine, ou par sélection de mutants viraux spontanés thermostables. Ces vaccins sont de production aisée, d'un coût relativement faible et injectables.

Ils induisent une protection rapide et de longue durée (Casselberry N.H., J.A.V.M.A., 152, 853-856, 1968). Ils présentent cependant un certain nombre d'inconvénients car ils possèdent une virulence résiduelle pour les vaches gestantes (avortements) et peuvent récupérer leur virulence originelle par réversion. De plus, la possibilité d'établissement d'une latence suite à leur injection n'est pas à écarter. Des souches thermosensibles ont été mises au point pour remédier à ces inconvénients mais elles nécessitent une administration par voie intranasale, ce qui est très peu pratique (Kahrs R.F. et al., J.A.V.M.A., 163, 437-441, 1973).
- Les vaccins préparés à base de virus inactivés, obtenus par traitement du virus pathogène par des agents physiques ou chimiques (Durand M. et al., Bull. Soc. Vet. Pr. 65, 193-204, 1981). La fabrication de ces vaccins est beaucoup plus coûteuse car elle nécessite de nombreux contrôles. Ces vaccins sont également moins efficaces que les vaccins vivants atténués et nécessitent plusieurs administrations successives en présence d'adjuvant pour induire une bonne protection.
- Les vaccins à sous-unités virales sont obtenus par purification partielle des glycoprotéines d'enveloppe responsables de l'immunité protectrice (Babiuk L.A. et al., Virology, 159, 57-66, 1987), après culture du virus BHV-1. D'une parfaite innocuité, ces vaccins sont, eux-aussi, d'un coût relativement élevé à cause de l'étape de purification, de la nécessité d'utiliser un adjuvant et des injections multiples requises pour induire une réponse immunitaire protectrice (Israël B.A. et al., Vaccine 6, 349-356, 1988).

Les vaccins couramment utilisés pour le contrôle de l'IBR sont donc loin d'être satisfaisants, soit parce qu'ils possèdent une virulence résiduelle, soit parce qu'ils sont peu pratiques à utiliser, soit enfin parce qu'ils sont relativement coûteux. D'autre part, le principal inconvénient des trois types de vaccins décrits ci-dessus est qu'ils ne permettent pas la distinction entre animaux infectés et animaux vaccinés, condition qui est absolument nécessaire pour mener une prophylaxie raisonnée, par vaccination et élimination conjointe des animaux infectés, en vue de l'éradication de l'IBR.
- Des souches vaccinales vivantes atténuées expérimentales ont été proposées comme solutions à ce problème. Elles ont été obtenues par recombinaison génétique par délétion du gène codant pour la thymidine kinase (Kit M. et al., European Patent Application n° 0316 658 A1, 1988). Ces souches ne permettent cependant pas de résoudre le problème causé par les animaux apparemment sains, mais porteurs de virus à l'état latent, qui représentent un réservoir potentiel de transmission de la maladie. Elles ne permettent pas non plus la distinction entre animaux vaccinés et animaux infectés.

D'autres vaccins à base de virus BHV-1 délétés au niveau des gènes codant pour la thymidine kinase et pour la glycoprotéine gIII (homologue de la glycoprotéine gC de HSV-1) ont été proposés (Kit M. et al., European Patent Application n° 0326 127 A2, 1989) afin de différencier les animaux vaccinés des animaux infectés. Les équivalents de cette glycoprotéine chez les herpèsvirus HSV-1 (gC) et PRV (gIII) se sont révélés être non essentiels pour la réplication virale in vitro (Holland T.C. et al., J. Virol., 52, 566-574, 1984 ; Kit S. et al., Am. J. Vet. Res., 48, 780-793, 1987), mais la glycoprotéine gIII participe de façon importante à l'induction d'anticorps neutralisants chez les animaux immunisés avec BHV-1 (Collins J.K. et al., J. Virol., 52, 403-409, 1984) ce qui pourraît être à l'origine d'une activité plus faible de ces vaccins sur le terrain.

L'impossibilité de distinguer les animaux infectés des animaux immunisés avec les vaccins commerciaux actuels est l'obstacle majeur à une politique d'éradication de la maladie dans les pays touchés. Accessoirement, cette situation est aussi un frein aux échanges internationaux d'animaux puisque de nombreux pays exigent que les bovins soient séronégatifs vis-à-vis de BHV-1 lors des importations. Toute politique d'éradication raisonnée fait appel à des mesures conjuguées de prophylaxie sanitaire (abattage) et de prophylaxie médicale (vaccination). Dans cette optique, un vaccin "idéal" est celui qui permettra de conférer au meilleur coût une immunité précoce, satisfaisante et durable, et qui, s'il est associé à un test de dépistage simple à mettre en oeuvre, permettra une distinction facile entre animaux vaccinés et animaux infectés.

Un virus BHV-1 mutant, délété dans un gène codant pour l'une ou plusieurs des glycoprotéines virales mineures ne participant pas de manière importante dans l'induction de la protection, et utilisé pour préparer un vaccin inactivé, atténué, ou sous-unités, répond à ces multiples objectifs.

### Le génome du BHV-1

La structure de son génome permet de classer le virus de la rhinotrachéite infectieuse bovine dans le groupe D des herpèsvirus auquel appartiennent également le virus de la maladie d'Aujeszky (PRV), les herpèsvirus équins de type 1 et 4, et celui de la varicelle (VZV) (Roizman B. et al., Arch. Virol., 123, 425-449, 1992). Le génome de BHV-1 est un ADN linéaire double brin d'environ 140 kb composé d'une séquence longue unique de 105 kb (U_{L}) et d'une séquence courte unique de 11 kb (U_{S}), cette dernière étant encadrée par deux régions répétées et inversées d'environ 12 kb (région répétée interne (IR) et région répétée terminale (TR). La partie U_{S} peut s'orienter dans l'un ou l'autre sens par rapport à la partie U_{L}, conférant au génome du BHV-1 la propriété d'exister sous deux formes isomériques (Wirth U.V. et al., J. Virol., 63, 4882-4889, 1989).

L'analyse des fragments de restriction après digestion de l'ADN viral par des endonucléases de restriction se révèle être un outil bien approprié pour la différenciation des souches BHV-1. Elle permet en effet de les classer en trois types associés à des manifestations cliniques précises (Bulach D.M. et Studdert M.J., Arch. Virol., 113, 17-34, 1990 ; Bratanich A.C. et al., J. Vet. Med., B38, 41-48, 1991 ; Engels M. et al., Arch. Virol., 67, 169-174, 1981).

Type BHV-1.1. Associé à l'IBR. La souche de référence isolée d'un cas d'IBR est la souche Cooper (Zucheck F. et Chow T.L., J.A.V.M.A., 139, 236-237, 1961).

Type BHV-1.2. Associé à l'IPV. La souche de référence isolée d'un cas de vulvovaginite est la souche K22 (Kendrick J.W. et al., Cornell Vet., 48, 458-495, 1958).

Type BHV-1.3. Associé à des symptômes neurologiques. La souche de référence est la souche BEHV N569 (French G.L., Austr. Vet., J., 38, 555-556, 1962).

Bien que BHV-1 code pour environ 50 à 100 gènes différents, très peu d'entre eux ont été localisés sur la carte physique du génome. Les gènes codant pour la thymidine kinase (Kit M. et Kit S. USA Patent Application Number 796.840, 1986 ; Mittal S.K. et Field H.J., J. Gen. Virol. 70, 901-918), l'ADN polymérase (Owen L.J. et Field H.J., Arch. Virol., 98, 27-38, 1988), la glycoprotéine gI (Misra V. et al., Virology, 166, 542-549, 1988 ; Whitbeck J.C. et al., J. Virol., 62, 3319-3327, 1988), la glycoprotéine gIII (Fitzpatrick D.R. et al., Viroloqy, 173, 46-57, 1989) et la glycoprotéine gIV (Tikoo S.K. et al., J. Virol., 64, 5132-5142, 1990) ont été localisés sur le qénome du BHV-1, séquencés, et analysés.

Un aspect de la présente invention comporte l'identification de la localisation et la séquence des gènes BHV-1 codant pour la glycoprotéine homologue de la glycoprotéine HSV-1 gI, et la séquence du gêne codant pour la protéine homologue de la protéine HSV-1 US9.

### Les glycoprotéines du BHV-1

Le génome du virus BHV-1 code pour au moins 40 à 48 protéines (Misra V. et al., J. Virol., 40, 367-378 ; Wirth U.V. et al., J. Virol., 65, 195-205, 1991). Une analyse électrophorétique, en PAGE-SDS, du virus BHV-1 purifié et radiomarqué révèle la présence de 25 à 33 polypeptides structuraux ayant une taille comprise entre 12 et 330 kDa. Parmi ces polypeptides structuraux, 11 ont été identifiés comme étant glycosylés (Misra V. et al., J. Virol., 40, 367-378, 1981) ; van Drunen Littel - van den Hurk S. et Babiuk L.A., J. Virol., 59, 401-410, 1986 ; van Drunen Littel - van den Hurk S. et al., Virology, 135, 466-479, 1984). Les glycoprotéines spécifiques du virus ont un rôle déterminant dans la relation hôte-virus car elles sont incorporées dans la membrane de la cellule infectée et deviennent finalement des constituants de l'enveloppe du virion. Elles ont par conséquent des rôles importants au niveau de la reconnaissance, de l'attachement et de la pénétration du virus dans les cellules permissives, dans la formation des syncytia et dans les différentes réponses du système immunitaire bovin à l'infection IBR, telles que la neutralisation du virus et la destruction immunologique des cellules infectées. A ce jour, quatre glycoprotéines ont été identifiées et caractérisées au niveau de la membrane des cellules infectées par BHV-1 et au niveau de l'enveloppe des virions. Elles sont dénommées gI, gII, gIII et gIV selon la nomenclature proposée pour la première fois en 1986 (van Drunen Littel - van den Hurk S. et Babiuk L.A., J. Virol., 59, 401-410, 1986).

Trois de ces glycoprotéines (gI, gIII et gIV) ont été plus particulièrement étudiées en raison de leur présence en quantités importantes au niveau de l'enveloppe du virion et parce que la majorité des anticorps anti-BHV-1 présents dans le sérum des animaux atteints d'IBR sont dirigés contre elles ; elles sont appelées pour cela glycoprotéines majeures (van Drunen Littel - van den Hurk S. et al., Virology, 135, 466-479, 1984) ; van Drunen Littel - van den Hurk S. et al., Virology, 144, 216-227, 1985 ; Marshall R.L. et al., J. Virol., 57, 745-753, 1986 ; Babiuk L.A. et al., Virology, 159, 57-66, 1987). Les gènes codant pour ces 3 glycoprotéines ont tous été isolés et séquencés. Ces trois glycoprotéines sont reconnues par des antisérums monospécifiques et par des anticorps monoclonaux neutralisants. Elles ont des tailles apparentes sur gel PAGE-SDS de 97 kDa (gIII), 77 kDa (gIV) et 130, 74 et 55 kDa (gI).

En plus de ces trois glycoprotéines majeures, on peut observer des glycoprotéines mineures d'environ 115 kDa, 64 kDa et 45 kDa de masse moléculaire. La glycoprotéine de 115 kDa, désignée gII, est spécifique du virus puisqu'elle est précipitée par des anticorps monoclonaux reconnaissant BHV-1 (van Drunen Littel - van den Hurk S. et al., Virology, 135, 466-479, 1984), mais jusqu'ici le gène codant pour cette glycoprotéine n'a pu être caractérisé.

Il a été démontré que, chez les herpèsvirus, plus d'une glycoprotéine est impliquée dans l'induction des anticorps neutralisants et des lymphocytes cytotoxiques qui aident à prévenir l'infection et à guérir d'une infection. Par exemple, les antisérums monospécifiques dirigés contre chacune des glycoprotéines gB, gC, gD et gE de HSV-1 sont capables de neutraliser le virus et de déclencher la lyse dépendante du complément des cellules infectées par le virus (Norrild B. et al., J. Virol., 32, 741-748, 1979). De la même manière, les anticorps monoclonaux dirigés contre les glycoprotéines gB, gC, gD et gF du virus herpès simplex de type 2 (HSV-2) déclenchent la lyse immunologique des cellules infectées (Balachandran N. et al., Infect. Imm., 37, 1132-1137). Par ailleurs, des anticorps neutralisants ont été produits à partir des qlycoprotéines gII, gIII et gp50 de PRV ; l'immunisation passive d'animaux avec des anticorps monoclonaux dirigés soit contre PRV gp50, soit contre PRV gIII les protège d'une infection avec le virus sauvage (Hampl H. et al., J. Virol., 52, 583-590, 1984 ; Ben Porat T. et al., Virology, 154, 325-334, 1986). De même, des anticorps monoclonaux dirigés contre les glycoproprotéines gp13, gp14 et gp17/18 de l'herpèsvirus équin de type 1 (EHV-1), inoculés à des hamsters, les protègent passivement contre une épreuve virulente avec EHV-1 (Stokes A. et al., J. Gen. Virol., 70, 1173-1183, 1989 ; Shimizu M. et al., Arch. Virol., 104, 169-174, 1989). En ce qui concerne le virus BHV-1, les glycoprotéines majeures gI, gIII et gIV induisent des niveaux élevés d'anticorps neutralisants chez les bovins et participent à la cytotoxicité cellulaire dépendant des anticorps.

Afin de développer un vaccin BHV-1 ayant un marqueur sérologique qui permette la distinction entre les animaux vaccinés et les animaux naturellement infectés, il a été nécessaire, dans la présente invention, d'identifier un gène codant pour une glycoprotéine de BHV-1 qui ne soit pas essentielle à la réplication du virus in vitro et qui n'intervienne pas de façon importante dans l'induction de la réponse immunitaire protectrice.

La glycoprotéine gI est l'homologue de la glycoprotéine gB de HSV-1 et de la glycoprotéine PRV gII (Whitbeck J.C. et al., J. Virol., 62, 3319-3327, 1988 ; Misra V. et al., Virology, 166, 542-549, 1988). De nombreuses données expérimentales indiquent que les gènes HSV-1 gB et PRV gII sont essentiels à la réplication du virus (Marlin S.D. et al., J. Virol., 53, 128-138, 1985 ; Lawrence W.C. et al., J. Virol., 60, 405-414, 1986 ; Bzik D.J. et al., Virology, 137, 185-190, 1984). Récemment, il a été montré que le produit du gène BHV-1 gI était lui-même indispensable à la réplication virale et qu'il pouvait fonctionnellement complémenter la glycoprotéine PRV gII dans des mutants PRV qII- (Kopp A. et Mettenleiter T.C., J. Virol., 66, 2754-2762, 1992). Il existe donc une grande convergence de résultats expérimentaux pour indiquer que les glycoprotéines "gB équivalentes" sont indispensables à la réplication des alphaherpèsvirus. En conséquence, le gène codant pour BHV-1 gI ne peut être un candidat pour servir de marqueur sérologique pour des vaccins BHV-1.

La glycoprotéine BHV-1 gIII est l'homologue de la glycoprotéine HSV-1 gC (Fitzpatrick D.R. et al., Viroloqy, 173, 44-57, 1989). Des mutants HSV-1 et HSV-2 incapables de synthétiser la glycoprotéine gC ont été isolés (Holland T.C. et al., J. Virol., 52, 566-574, 1984 ; Johnson D.C. et al., J. Virol., 58, 36-42, 1986). De même, un mutant PRV délété pour les gènes tk et gIII a été isolé (Kit S. et al., Am. J. Vet. Res., 48, 780-793, 1987). Se basant sur le même principe, les mêmes auteurs ont obtenu un virus BHV-1 mutant délété pour les gènes tk et gIII qui permet théoriquement de distinguer les bovins vaccinés des bovins infectés natùrellement par BHV-1 (Kit M. et al., European Patent Application Number 0316 658, 1988). Ce brevet n'a pas d'interférence avec la présente invention.

Bien que non indispensable à la réplication, la glycoprotéine BHV-1 gIII est toutefois reconnue majoritairement par des anticorps monoclonaux neutralisants (Ludwig G.V. et Letchworth G.J. III, J. Virol., 61, 3292-3294, 1987 ; van Drunen Littel - van den Hurk S. et al., Virology, 135, 466-479, 1984 ; van Drunen Littel - van den Hurk S. et al., Vaccine, 8, 358-368, 1990) et permet une protection efficace des animaux immunisés (Babiuk L.A. et al., Virology, 159, 57-66, 1987). Cette glycoprotéine, comme toutes les autres glycoprotéines "gC équivalentes" chez les alphaherpèsvirus, a donc un rôle important dans l'induction d'une immunité protectrice vis-à-vis de BHV-1. Le gène codant pour BHV-1 gIII n'est donc pas non plus un bon candidat pour obtenir un virus BHV-1 délété qui servira à la préparation de vaccins.

La glycoprotéine BHV-1 gIV est l'homologue de la glycoprotéine HSV-1 gD (Tikoo S.K et al., J. Virol., 64, 5132-5142, 1990). Comme les glycoprotéines gI et gIII, elle a un rôle majeur dans l'induction de la réponse immunitaire anti-BHV-1 protectrice chez les bovins infectés. Le gène codant pour BHV-1 gIV se trouve sur la partie U_{S} du génome, comme c'est le cas pour les autres glycoprotéines "gD équivalentes" déjà caractérisées chez HSV-1, PRV et EHV-1 (Tikoo S.K. et al., J. Virol., 64, 5132-5142, 1990 ; Petrovskis E.A. et al., J. Virol., 59, 216-223, 1986 ; Mc Geoch D.J. et al., J. Mol. Biol. 181, 1-13, 1985 ; Audonnet J.C. et al., J. Gen. Virol., 71, 2969-2978 , 1990). Les protéines HSV-1 gD et PRV gp50 seraient indispensables à une étape précoce du processus de pénétration du virus dans les cellules (Sodora et al., J. Virol., 65, 4432-4441, 1991 ; Rauh I. et Mettenleiter T., J. Virol., 65, 5348-5356, 1991). Son essentialité pour la réplication de BHV-1 n'a pas encore été démontrée, contrairement à HSV-1 gD (Longnecker R. et Roizman B., Science, 236, 573-576, 1987). La glycoprotéine gIV joue par ailleurs un rôle majeur dans l'immunogénicité virale. La majorité des anticorps monoclonaux dérivés d'une fusion cellulaire utilisant des splénocytes de souris immunisée avec du virus BHV-1 entier sont en effet dirigés contre la glycoprotéine gIV (Marshall R.L. et al., Virology, 165, 338-347, 1988 ; Chang L.W.S. et al., Arch. Virol., 88, 203-215, 1986). De plus, ces anticorps possèdent une activité neutralisante, même en absence de complément (van Drunen Littel - van den Hurk S. et al., J. Clin. Microbiol., 23, 274-282, 1986) et sont impliqués dans la lyse cellulaire dépendant des anticorps (Ludwig G.V. et Letchworth G.J. III, J. Virol., 61, 3292-3294, 1987).

Enfin, il est possible de protéger des veaux contre une épreuve BHV-i en les immunisant avec la glycoprotéine gIV purifiée (Babiuk L.A. et al., Virology, 159, 57-66, 1987). Il apparaît donc que la glycoprotéine gIV ne constitue pas, elle aussi, une bonne cible pour l'obtention de mutants BHV-1 délétés.

On peut rechercher en fait des gènes de glycoprotéines non essentielles à la réplication de BHV-1 in vitro, et pouvant être utilisées comme marqueurs sérologiques, parmi les gènes localisés sur la partie U_{S} du génome de BHV-1.

Dans la présente invention, il a été possible, pour la première fois, d'identifier, de cloner et de séquencer les gènes BHV-1 codant pour les protéines homologues de HSV-1 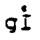 et HSV-1 US9. De plus, dans la présente invention, il a été possible de montrer pour la première fois que le gène codant pour la glycoprotéine BHV-1 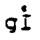 n'était pas indispensable pour la réplication virale in vitro. En conséquence, il a été possible pour la première fois de créer des mutations par recombinaison génétique dans le gène BHV-1 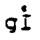 . Comme pour les mutations par délétion et/ou par insertion dans le gêne gE, ces virus mutants ne peuvent plus synthétiser les polypeptides antigéniques codés par le gènes 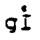 . En conséquence, les animaux immunisés avec des vaccins préparés à partir des virus mutants décrits par la présente invention ne feront pas d'anticorps contre la glycoprotéine BHV-1 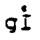 , ou 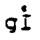 et gE s'ils sont délétés dans les deux gènes.

Les animaux vaccinés pourront donc être distingués des animaux infectés par les souches BHV-1 du terrain, ce qui permet d'envisager l'éradication de la maladie IBR par l'application d'une prophylaxie médicale gui n'interfère pas avec les mesures de prophylaxie sanitaire L'expression des glycoprotéines codées par les gènes BHV-1 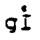 et BHV-1 gE dans un système d'expression approprié permet de produire les antigènes nécessaires à la détection des animaux infectés.

Les vaccins peuvent être des vaccins vivants ou inactivés à base de virus BHV-1 délété au moins dans le gène codant pour 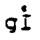 . De préférence, le virus utilisé peut comporter une délétion supplémentaire dans le gène codant pour la glycoprotéine gE. On préfére particulièrement les vaccins à base de glycoprotéines d'enveloppe et/ou protéines ou polypeptides purifiés obtenus par purification à partir du virus BHV-1 délété. Le procédé permettant l'obtention de ces sous-unités purifiées est bien connu (Babiuk L.A. et al., Virology, 159, 57-66, 1987).

### Exemple 1

### Clonage et séquengage partiel de la réqion BHV-1 U_{S}

### A. Virus et culture du virus

Le virus utilisé comme virus parental est la souche ST de BHV-1, souche de production pour les vaccins de RHONE MERIEUX. Cette souche a été isolée en 1970 sur un bovin atteint de rhinotrachéite infectieuse bovine.

Pour les expériences de transfection, le virus a été cultivé sur cellules IPB3 (lignée semi-établie de poumon de foetus bovin). Pour l'isolement des virus recombinants, les virus ont été cultivés sur cellules RV (lignée semi-établie de rein de veau). Toutes les cellules ont été cultivées en milieu de Eagle modifié par Dulbecco (DMEM) contenant 5% de sérum de veau foetal.

### B. Purification de BHV-1 et extraction de l'ADN viral

L'ADN viral purifié nécessaire aux expériences de clonaqe et aux analyses en Southern Blot a été préparé à partir de virus purifié. La purification du virus se fait à partir d'une culture infectée présentant 100% d'effet cytopathogène. Le surnageant de culture est clarifié par centrifugation à 3 800 q pendant 15 mn pour éliminer les débris cellulaires. Les virions présents dans le surnageant sont alors concentrés par sédimentation à 236 000 g pendant 1 heure, repris en tampon TBSA (Tris 10mM pH 7,5 ; NaCl 136mM, KCl 2,6mM ; Mg Cl₂ 0mM ; CaCl₂ 1,8mM ; albumine sérique bovine 1%) et déposés sur un qradient de saccharose en couches de 30% à 60% (poids/poids) en TBSA. Après ultracentrifugation isopycnique (88 000 g, 18 heures), la bande virale est récupérée et diluée dans 4 volumes de TBSA. Les virions purifiés sont alors concentrés par une nouvelle ultracentrifugation (272 000 g, 1 heure) et repris dans un faible volume de TBSA. L'extraction de l'ADN viral à partir des virus purifiés est ensuite réalisée selon une technique standard (Maniatis T. et al., Molecular cloning : a laboratory manual, Cold Sprinq Harbor Laboratory, Cold Spring Harbor, NY, 1982).

L'ADN viral infectieux nécessaire aux expériences de transfection est préparé selon une technique décrite par Hirt (Hirt B., J. Mol. Biol., 26, 365-369, 1967). Lorsque les cellules infectées présentent un début d'effet cytopathogène, le milieu de culture est élimine et les cellules sont mises en contact pendant quelques minutes avec une solution de TE (Tris 10mM pH 8,0, EDTA 1mM) à 1% de SDS à raison de 1 ml pour 5.10⁶ à 10⁷ cellules. De la protéinase K est alors ajoutée à cette solution, de façon à obtenir une concentration finale de 200 µg/ml. Après incubation d'une heure à 37°C, une solution NaCl 5M est aioutée de manière à obtenir une concentration finale 1M NaCl et le mélange est placé une nuit à 0°C. Une centrifugation (12 000 q, 30 mn) permet de récolter le surnageant qui est alors incubé en présence de protéinase K 200 µg/ml final à 37°C pendant une heure. Après deux extractions au phénol-chloroforme, la phase aqueuse est prélevée et précipitée par 2.5 volumes d'éthanol absolu pendant une nuit à -20°C. Après centrifugation, le culot est repris en TE.

### C. Clonage du fragment EcoRI 16,4 kb

Le fragment EcoRI 16,4 kb contenant la totalité de la région U_{S} et une partie des régions répétées de la souche BHV-1 ST a été cloné dans le vecteur pBluescript SK+ (Stratagène) digéré par EcoRI. Des bactéries compétentes Escherichia coli NM 522 ont été transformées avec le produit de la ligature et les clones obtenus ont été caractérisés par analyse de restriction selon des techniques standards (Maniatis et al., 1982). Le clone contenant le fragment BHV-1 EcoRI 16,4 kb a été désigné pBHV001.

D. Séquence du fragment BHV-1 U_{S} codant pour les glycoprotéines homologues de HSV-1 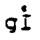 . HSV-1 gE et pour la protéine homoloque d'HSV-1 US9

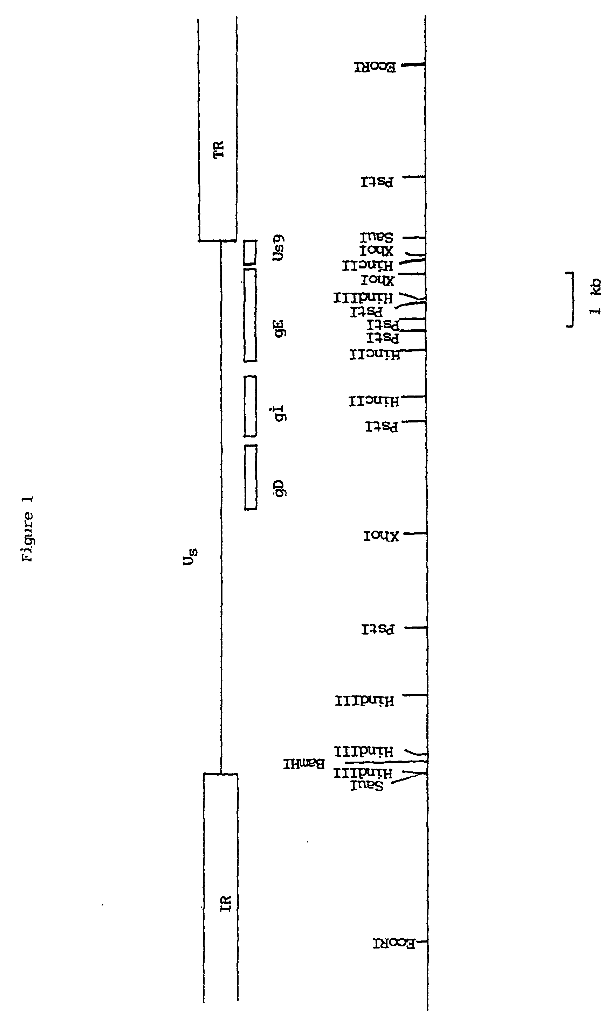

La carte de restriction du fragment EcoRI de 16,4 kb a été établie avec les enzymes BamHI, HincII, HindIII, PstI, SauI et XhoI (figure 1) et la séquence d'un fragment de 4190 pb (figure 2) a été établie à partir de sous-clones dans le vecteur pBluescript SK+ générés à partir de digestions du fragment EcoRI de 16,4 kb avec diverses enzymes de restriction. Les personnes expertes dans l'art reconnaîtront qu'il y a de multiples façons de générer ces sous-clones. La séquence complète des deux brins du fragment de 4 190 pb a été obtenue en utilisant l'enzyme modifiée T7 Sequenase (U.S Biochemical Corporation) (Tabor S. et Richardson C.C., Proc. Nat. Acad. Sci., USA, 84, 4767-4771, 1987). Des réactions standards de termination de chaînes (Sanger F. et al., Proc. Nat. Acad. Sci. USA, 74, 5463-5467, 1977) ont été réalisées sur des matrices double brin préalablement dénaturées dans une solution NaOH 0,4M (Hattori M. et Sakaki Y., Anal. Biochem., 152, 1015-1026, 1986). Les oligonucléotides M13 "forward" et réverse ont été utilisés pour obtenir les séquences initiales à chaque extrémité des sous-clones. Des oligonucléotides de synthèse, synthétisés selon des techniques standards (Applied biosystems 381 A) ont été utilisés pour les réactions de séquence suivantes. Le logiciel d'analyse de séquence PC/GENE (Intelligenetics) a été utilisé pour toutes les analyses des séquences nucléotidiques et protéiques.

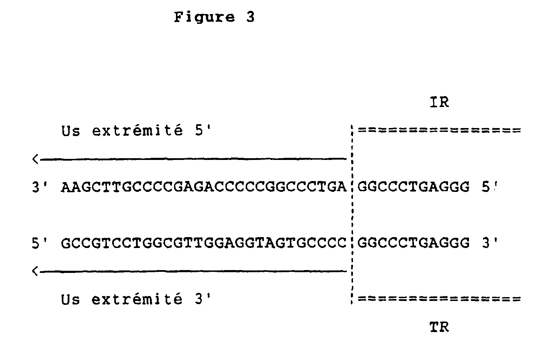

La séquence nucléotidique de 4 190 pb présentée dans cette invention (figure 2) est localisée principalement sur la région U_{S} du génome de la souche BHV-1 ST mais comprend la jonction U_{S}/TR et une petite partie (334 pb) de la région répétée terminale. La jonction U_{S}/régions répétées a été identifiée par comparaison des séquences obtenues à partir de sous-clones contenant respectivement les régions 5' et 3' de la région BHV-1 ST U_{S} (figure 3).

Il est entendu que. relativement à la séquence nucléotidique de l'ADN génomique de BHV-1, des variations naturelles peuvent exister selon les différentes souches. Ces variations peuvent être des délétions, des substitutions, des insertions, des inversions ou des additions d'un ou plusieurs nucléotides, qui peuvent éventuellement modifier la position d'un ou plusieurs sites de restriction, produisant ainsi une carte de restriction proche de celle présentée dans la figure 1.

La région séquencée contient 3 cadres ouverts de lecture complets codant Potentiellement pour des protéines. Les séquences d'acides aminés déduites de ces cadres ouverts de lecture sont indiquées en regard de la séquence présentée dans la figure 2.

Ces séquences protéiques ont été comparées en homologie avec les protéines codées par les régions U_{S} d'autres alphaherpèsvirus. Les résultats de cette comparaison sont présentés dans le tableau I.

Il apparaît que les meilleurs scores pour le premier cadre ouvert de lecture sont obtenus avec les glycoprotéines " 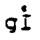 équivalentes" codées par EHV-1, PRV et HSV-1 ; ce premier cadre ouvert de lecture a donc été désigné BHV-1 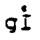 . Les meilleurs scores pour le deuxième cadre ouvert de lecture sont obtenus avec les glycoorotéines "gE équivalentes" codées par EHV-1, PRV et HSV-1 ; ce deuxième cadre ouvert de lecture a donc été désigné BHV-1 gE. Enfin, les meilleurs scores pour le troisième cadre ouvert de lecture sont obtenus avec les protéines "US9 équivalentes" codées par PRV, VZV et HSV-1 ; ce troisième cadre ouvert de lecture a donc été désigné BHV-1 US9.

### Exemple 2

### Construction du plasmide de délétion pBHV 008

Le plasmide pBHV001 contenant le fragment EcoRI - EcoRI de 16,4 kb (figure 1) a été digéré par EcoRI et HindIII pour libérer le fragment HindIII - EcoRI d'environ 4,4 kb. Ce fragment a été cloné dans le vecteur pBluescript SK+, préalablement digéré par EcoRI et HindIII pour générer le plasmide pBHV003.

Le plasmide pBHV003 a été digéré par SauI, réparé par la polymérase Klenow, puis digéré par PstI pour libérer le fragment PstI-bout franc de 1,1 kb qui a alors été cloné dans le vecteur pBluescript SK+, préalablement digéré par PstI et SmaI, pour générer le plasmide pBHV005 contenant la région 3' flanquante du plasmide de délétion.

Le plasmide pBHV001 a été digéré par HindIII pour libérer le fragment HindIII - HindIII de 7,5 kb (figure 1). Ce fragment a été cloné dans le vecteur pBluescript SK+ préalablement digéré par HindIII et traité par la phosphatase alcaline pour générer le plasmide pBHV004.

Le plasmide pBHV004 a été digéré par PstI pour obtenir un fragment de 3,8 kb qui a été cloné dans le site PstI de pBluescript SK+ pour générer le plasmide BHV006. Le plasmide pBHV006 a été digéré par XhoI, réparé par la polymérase Klenow, et digéré par SpeI pour libérer finalement un fragment SpeI - bout franc de 2,1 kb. Ce fragment a alors été cloné dans le plasmide pBHV005 préalablement digéré par NotI, réparé par la polymérase Klenow, et digéré par SpeI, pour générer le plasmide pBHV 007 qui comporte une délétion allant du nucléotide 430 au nucléotide 3 861 sur la séquence présentée sur la fiqure 2.

Pour faciliter le travail d'isolement et de purification du virus recombinant, une cassette d'expression du gène Eco gpt placé sous le contrôle du promoteur précoce SV40 a été insérée au niveau de la délétion. Cette cassette d'expression est donnée ici seulement comme un exemple de marqueur utilisable pour détecter les recombinants délétés. Les personnes de l'art reconnaîtront qu'il existe de nombreux autres exemples de gènes pouvant être utilisés comme traceurs et nous ne limitons pas cette partie de l'invention à un type unique de gène traceur ou à un type unique de promoteur pour contrôler l'expression de ce gène traceur. Brièvement, le plasmide pMAM (Clontech) a été digéré par BamHI pour libérer la cassette SV40 Eco gpt contenue dans ce plasmide. Cette cassette a alors été clonée dans le plasmide pBHV007, préalablement digéré par BamHI, pour générer le plasmide pBHV008 qui contient la cassette d'expression Eco qpt en lieu et place de la délétion précédemment décrite, encadrée en 5' et 3' par les séquences flanquantes nécessaires à la recombinaison homologue dans la région US au génome du virus BHV-1 ST.

### Exemple 3

### Construction des plasmides de délétion pBHV010 et pBHV011

Le plasmide pBHV003 a été digéré par PvuI, traité avec la polymérase Klenow, et digéré par HindIII pour libérer le fragment (Bout franc) - Hind III de 3 kb.

Ce fragment a été cloné dans un vecteur pBR322 muté au niveau du site de restriction unique Sali et digéré par EcoRV et HindIII pour générer le plasmide pBHV009. Le plasmide pBHV009 a été digéré par Sali pour déléter le fragment Sal I - Sal I de 840 pb (positions 873 --> 1 713 sur la séquence de la figure 2) et isoler le fragment Sal I - Sal I de 6 360 pb (fragment A). Le plasmide pSVß (Clontech) contenant une cassette SV40 - lacZ a été digéré par EcoRI et Sali pour libérer le fragment EcoRI - Sali de 3 855 pb (cassette SV40 - lacZ (fragment B). Les fraoments A et B ont alors été réparés avec la polymérase Klenow et ligaturés ensemble pour générer les plasmides pBHV010 et pBHV011 qui correspondent respectivement aux orientations droite et gauche de la cassette SV40 - lacZ relativement aux séquences BHV-1 flanguantes.

Exemple 4

Obtention de recombinants BHV-1 délétés dans 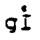 , gE et US9

### A. Transfections

Toutes les expériences de transfection ont été réalisées selon une technique standard de précipitation au phosphate de calcium (kit "calcium phosphate transfection system" commercialisé par Gibco-BRL). Le précipité réalisé avec 5 µg d'ADN viral et une quantité variable d'ADN plasmidique (pBHV008) (5 à 20 µg) à l'aide du kit est ajouté doucement à des cellules IPB3 en culture depuis 24 heures en flacon de 25 cm². Après une nuit à 37°C, les cellules sont rincées avec du milieu sans sérum, puis incubées d'ans du DMEM 1% SVF jusqu'à l'apparition d'ECP. Lorsque celui-ci est généralisé, le surnageant de cotransfection est aliquoté et conservé à -80°C.

La cotransfection peut également être réalisée à l'aide des autres techniques connues par les personnes de l'art, en particulier selon la technique des liposomes, effectuée avec le kit "lipofectine" de Gibco-BRL.

### B. Isolement et purification des virus recombinants

L'isolement et la purification des virus recombinants ont été réalisés selon la technique de clonage sous agarose décrite ci-dessous. Des cellules RV sont cultivées en boites de Petri (Corning 4,5 cm de diamètre) dans du milieu DMEM à 10% de SVF jusqu'à ce que les tapis cellulaires soient bien établis. Le milieu de culture est alors éliminé et 1 ml de surnageant issu de la transfection précédemment décrite, et dilué de façon à obtenir des plages isolées, est adsorbé sur les cellules pendant 1 heure à 37°C. Le surnageant de transfection est éliminé et remplacé par 5 ml de milieu DMEM à 1% d'agarose, maintenu en surfusion à 37°C. Lorsque l'agarose est solidifiée, les boîtes sont incubées 48 à 72 heures à 37°C dans une étuve à CO₂ jusqu'à apparition de plages. Les plages, repérées à l'oeil nu ou au microscope, sont prélevées à travers l'agarose à l'aide d'une micropipette et chaque prélèvement, après dissociation en milieu DMEM, est mis en culture dans une cupule, contenant des cellules RV établies en tapis et cultivées en DMEM 10% SVF, sur une plaque 96 cupules (Falcon).

Une fois amplifiée, la progénie de chaque plage est analysée à partir du surnageant de culture de chaque cupule. La moitié du volume de chaque surnageant est analysée selon une technique standard de dot blot avec une sonde Eco gpt ou avec la sonde appropriée si un autre gène traceur est utilisé. L'autre moitié du surnageant est conservée à -80°C et sert, après sonication, à un nouveau cycle de clonage sous agarose en boites de Petri comme précédemment décrit. Le virus recombinant a été purifié par 4 cycles successifs de clonage/hybridation et désigné BHV-1 A11.

Lorsque la transfection est réalisée avec un plasmide d'insertion contenant une cassette d'expression SV40-lacZ (plasmides d'insertion pBHV010 ou pBHV011), la détection et l'isolement des recombinants est réalisée comme suit :
L'étape de transfection est réalisée comme décrite précédemment. Le surnageant de transfection est éliminé et remplacé par 5 ml de milieu DMEM à 1% d'agarose. Lorsque l'agarose est solidifiée, les boites sont incubées 48 à 72 heures à 37°C dans une étuve à CO₂, jusqu'à apparition de plages. On ajoute alors sur l'agar une solution de X-gal (500 µg/ml) dans du milieu DMEM à 1% d'agarose, maintenu en surfusion à 37°C. Les boîtes de Petri sont alors incubées à 37°C dans une étuve à CO₂ pendant 3 à 4 heures et les plages bleues sont prélevées à travers l'agarose à l'aide d'une micropipette. Chaque plage prélevée est dissociée dans du milieu DMEM et des dilutions de chaque plage sont adsorbées comme précédemment sur des cellules RV. Quatre cycles de purification sont nécessaires pour obtenir un virus recombinant à 100% de pureté.

### C. Analyse en Southern blot de l'ADN du recombiant BHV-1 A11

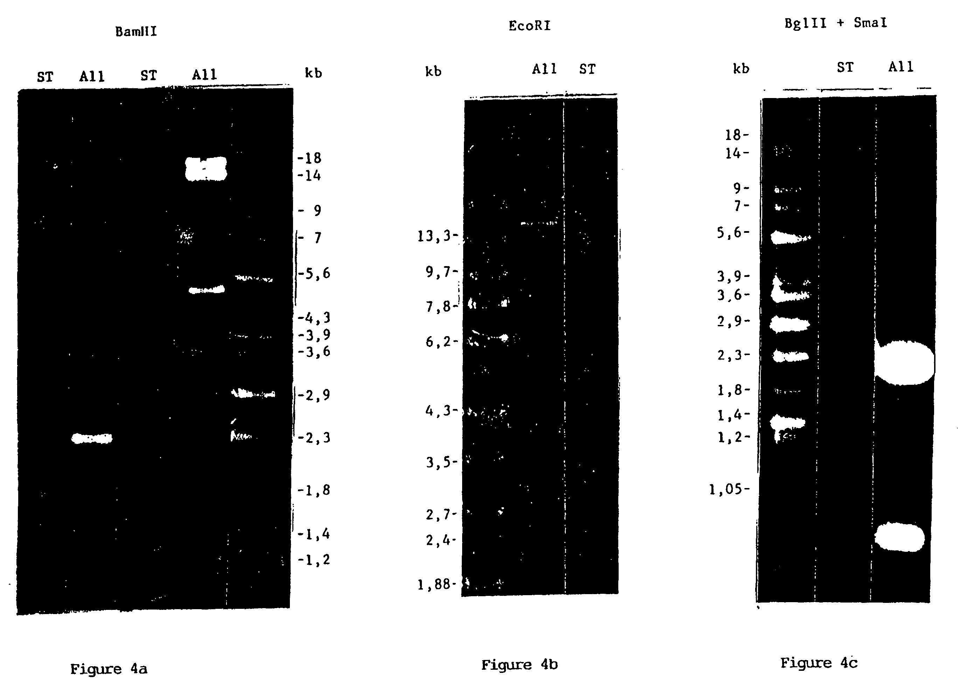

Pour étudier si la recombinaison était survenue de manière homologue sur le fragment U_{S} de BHV-1, l'ADN du virus recombinant "A 11" a été analysé en Southern blot (figures 4a, 4b et 4c). Les blots ont été sondés avec le fragment 2,1 kb Bam HI - Bam HI, qui correspond à la cassette SV40 - Eco gpt insérée dans le plasmide de délétion, et avec le vecteur 2,9 kb pBluescript. Pour chaque blot, l'ADN du virus parental BHV-1 ST et l'ADN du virus recombinant BHV-1 A11 (1 µg de chaque échantillon) ont été digérés avec les enzymes suivantes avant d'être déposés sur gel : Bam HI, Eco RI, Hind III, Bgl II + Sma I. Un témoin cellules non infectées a été inclus dans chaque blot.

Les résultats de ces hybridations ont montré que la recombinaison était effectivement survenue au niveau du site choisi.
- La sonde Eco gpt hybride sur un fragment Bam HI de 2,1 kb, ce qui indique que la cassette SV 40 Eco gpt a été intégrée dans le génome du virus A 11. Elle hybride sur un fragment Eco RI de 15 kb, ce qui correspond à la taille du fragment EcoRI de 16,4 kb, délété de 3,5 kb, et ayant inséré la cassette SV 40 - Eco gpt 2,1 kb. Elle hybride aussi sur des fragments Hind III de 18 kb, 14 kb et 5,2 kb, ce qui indique que la recombinaison a bien eu lieu au site choisi et que les deux formes isomériques du génome recombinant sont présentes. Elle hybride enfin sur deux fragments Bgl II - Sma I de 2,3 kb et 0,7 kb qui correspondent aux tailles théoriques attendues chez le virus recombinant.
- La sonde pBluescript n'hybride sur aucun fragment Bam HI, EcoRI, HindIII, ou BglII + SmaI du virus recombinant A 11, ce qui indique que la recombinaison s'est faite sans intégration de séquences du vecteur dans le génome du virus (non montré).

### Exemple 5

### Clonage et expression de la glycoprotéine BHV-1 qE dans un vecteur baculovirus

### A. Matériels

Le plasmide pBHV001 a servi de source d'ADN pour isoler le gène BHV-1 gE. Le plasmide pAcRP23 a été utilisé pour construire le plasmide de recombinaison (Luckow V.A et Summers M.D., Viroloqy, 170, 31-99, 1989). Le baculovirus Autographa californica Ac NPV (Summers M.D. et Smith G.E., A manual of methods for baculovirus vectors and insect cell culture procedures, Texas Agricultural Experimental Station bulletin n° 1555. Texas Aqricultural Experimental Station, College Station, Texas) a été utilisé comme virus parental pour les expériences de recombinaison. La lignée cellulaire Sf 21 (cellules dérivées de Spodoptera frugiperda) a été utilisée pour les cultures de virus parental et des virus recombinants. Les cellules ont été cultivées en milieu TC100 (Flow Labs).

### B. Construction du plasmide de recombinaison pIL003

Le fragment Sali - SalI de 840 pb (coordonnées 873-1713 sur la séquence de la figure 2) a été cloné dans le vecteur M13 mp18 et mutagénisé par la méthode d'Eckstein (Eckstein F. et al., Nucl. Acids Res., 16, 791-802, 1988) avec l'oligonucléotide IL001 (SEQ ID n° 2 : 5' GGCATTTGGATCCAATGCAACCCAC 3') pour introduire un site Bam HI juste en amont de l'ATG du gène BHV-1 gE. Le fragment muté a été digéré par SalI et BamHI pour libérer un fragment BamHI - SalI de 120 pb (fragment A). Le plasmide pBHV001 contenant le fragment BHV-1 EcoRI de 16,4 kb a été digéré par SalI et AsuII pour libérer le fragment Sali - AsuII de 1 715 pb (coordonnées 1 713 --> 3 425 sur la séquence de la figure 2). Ce fragment a été ensuite digéré par HindIII pour obtenir les fragments SalI - HindIII de 1 000 pb (fragment B) et HindIII - AsuII de 715 pb (fragment C). Les fragments A et B ont été ligaturés dans le vecteur pBluescript SK+ préalablement digéré par BamHI et HindIII pour générer le plasmide pIL001. Le fragment C et un adapteur synthétique AsuII - EcoRV - BamHI ont été ligaturés dans le vecteur pBluescript SK+ préalablement digéré par BamHI et HindIII pour générer le plasmide pIL002. Le plasmide pIL001 a été digéré avec BamHI et HindIII pour libérer le fragment BamHI - HindIII de 1 100 pb (fragment D). Le plasmide pIL002 a été digéré avec BamHI et HindIII pour libérer le fragment BamHI - HindIII de 730 pb (fragment E). Les fragments D et E ont alors été ligaturés dans le vecteur pAcRP23, préalablement digéré par BamHI et traité par la phosphatase alcaline, pour générer le plasmide pIL003. Le plasmide pIL003 contient le gène BHV-1 gE sous le contrôle du promoteur du gène de la polyhédrine et les séquences 5' et 3' flanquantes permettant la recombinaison homologue dans Autographa californica Ac NPV.

### C. Transfection

Les recombinants baculovirus ont été générés par cotransfection d'1 µg d'ADN d'Ac NPV et de 2 µg d'ADN de pIL003 dans des cellules de S. frugiperda selon la technique de précipitation au phosphate de calcium. Après 4 heures de contact à température ambiante, les cellules sont rincées avec du milieu TC100 et cultivées pendant 3 jours à 28°C. Les surnageants sont alors récupérés pour être inoculés dans les boites de Petri qui serviront à l'isolement des recombinants.

### D. Isolement et purification du virus recombinant

Des dilutions du surnageant de cotransfection sont adsorbées pendant 1 heure à température ambiante sur des cellules Sf21 cultivées en boites de Petri. Le surnageant est alors éliminé et remplacé par du milieu de culture à 2% d'agarose en surfusion. Lorsque la gélose est solidifiée, 1 ml de milieu est ajouté sur la couche d'agar, et les cellules sont incubées à 28°C pendant 3 à 4 jours. Les boites de Petri sont alors colorées au rouge neutre pendant 1 heure. Les plages polyhédrine négatives sont prélevées avec une micropipette, mélangées avec du milieu de culture et vortexées. Des dilutions de chaque plage sont alors préparées et adsorbées comme précédemment sur des cellules S. frugiperda. Trois cycles de purification ont été réalisés pour obtenir un virus recombinant à 100% de pureté.

Le baculovirus recombinant obtenu à partir de la transfection avec le plasmide pIL003 a été désigné rBAC001.

L'invention qui vient d'être décrite peut faire l'objet de nombreuses variantes spontanément accessibles à l'homme de l'art.

Ainsi, au lieu de cloner la région d'insertion par le procédé décrit, on peut la localiser par hybridation ou l'amplifier au moyen de sondes fabriquées de façon classique à partir des séquences nucléotidiques SEQ ID No : 1 ou 2 et l'insérer ensuite dans les plasmides ou vecteurs usuels, par exemple dans le cas où l'on part d'une souche BVH naturelle comportant des variations dans les sites de restriction utilisés.

De même, la région d'insertion selon l'invention enqlobe toutes les variantes d'origine naturelle ou non, que continuent de permettre à l'homme de l'art, d'assurer les délétions ou insertions selon l'invention.

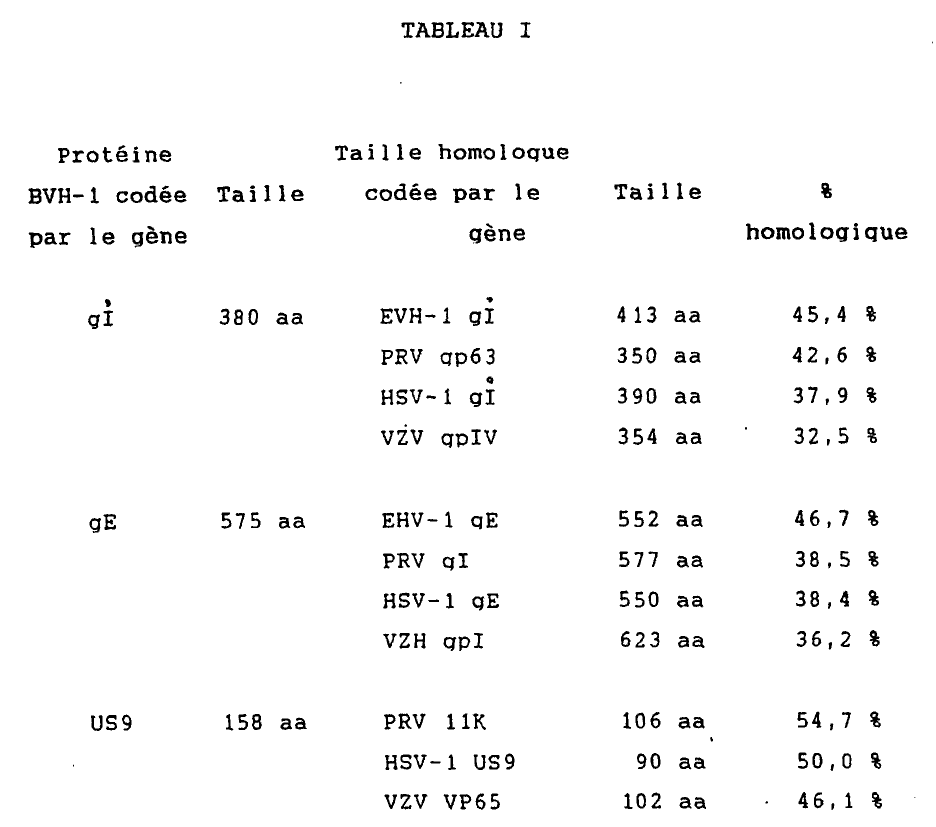

## Revendications

1. Procédé de production de protéine 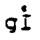 de BHV-1 dans lequel on fait exprimer par un baculovirus recombinant, un fragment d'ADN comprenant la séquence nucléotidique 172 à 1311 apparaissant à la SEQ ID NO : 1.

2. Procédé de production d'une protéine gE de BHV-1, dans lequel on fait exprimer par un baculovirus recombinant, un fragment d'ADN comprenant la séquence nucléotidique 1594 à 3318 apparaissant à la SEQ ID NO : 1.

3. Procédé selon la revendication 1 ou 2, **caractérisé en ce que** le baculovirus est Autographa californica.

4. Procédé selon l'une des revendications 1 à 3, **caractérisé en ce que** l'on cultive le baculovirus recombinant sur une lignée de cellules dérivées de Spodoptera frugiperda.

5. Procédé selon la revendication 4, **caractérisé en ce que** la lignée de cellules est la lignée Sf21.

6. Protéine isolée 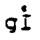 ou gE de BHV-1 susceptible d'être produite par le procédé selon l'une quelconque des revendications 1 à 5.

7. Procédé de production d'anticorps dirigés contre 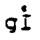 ou gE de BHV-1, comprenant l'administration à un hôte approprié d'une quantité immunogène d'une protéine selon la revendication 6.

8. Anticorps isolé dirigé contre 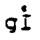 ou gE de BHV-1 susceptible d'être obtenu par la mise en oeuvre du procédé selon la revendication 7.

## Patentansprüche

1. Verfahren zur Herstellung des Proteins gI des BHV-1, bei dem man mit'einem rekombinanten Baculovirus ein Fragment von ADN exprimiert, das die in SEQ ID NO: 1 gezeigte Nukleotidsequenz 172 bis 1311 umfasst.

2. Verfahren zur Herstellung eines Proteins gE des BHV-1, bei dem man mit einem rekombinanten Baculovirus ein Fragment von ADN exprimiert, das die in SEQ ID NO: 1 gezeigte Nukleotidsequenz 1594 bis 3318 umfasst.

3. Verfahren nach Anspruch 1 oder 2, **dadurch gekennzeichnet, dass** der Baculovirus Autographa California ist.

4. Verfahren nach einem der Ansprüche 1 bis 3, **dadurch gekennzeichnet, dass** man den rekombinanten Baculovirus mit einer Zell-Linie kultiviert, die von Spodoptera frugiperda abgeleitet ist.

5. Verfahren nach Anspruch 4, **dadurch gekennzeichnet, dass** die Zell-Linie die Linie Sf21 ist.

6. Isoliertes Protein gI oder gE, das nach dem Verfahren nach einem der Ansprüche 1 bis 5 erhältlich ist.

7. Verfahren zur Herstellung eines gegen gI oder gE aus BHV-1 gerichteten Antikörpers, das umfasst, das man einem geeigneten Wirt eine immunogene Menge eines Proteins nach Anspruch 6 verabreicht.

8. Isolierter gegen gI oder gE aus BHV-1 gerichteten Antikörper, erhältlich mittels des Verfahrens nach Anspruch 7.

## Claims

1. A process for the production of protein gI of BHV-1 wherein a DNA fragment comprising the nucleotidic sequence 172 to 1311 appearing at SEQ ID NO:1 is expressed by a recombinant baculovirus.

2. A process for the production of a protein gE of BHV-1 wherein a DNA fragment comprising the nucleotidic sequence 1594 to 3318 appearing at SEQ ID NO:1 is expressed by a recombinant baculovirus.

3. A process according to claim 2 or claim 2 **characterised in that** the baculovirus is Autographa californica.

4. A process according to one of claims 1 to 3 **characterised by** cultivating the recombinant baculovirus on a line of cells derived from Spodoptera frugiperda.

5. A process according to claim 4 **characterised in that** the line of cells is the line Sf21.

6. An isolated protein gI or gE of BHV-1 capable of being produced by the process according to any one of claims 1 to 5.

7. A process for the production of antibodies directed against gI or gE of BHV-1 comprising the administration to a suitable host of an immunogenic quantity of a protein according to claim 6.

8. An isolated antibody directed against gI or gE of BHV-1 capable of being obtained by carrying out the process according to claim 7.
